Europäisches Patentamt

European Patent Office

Office européen des brevets

(11) Publication number: **0 396 402**
**A2**

## (12) EUROPEAN PATENT APPLICATION

(21) Application number: **90304784.3**

(22) Date of filing: **02.05.90**

(51) Int. Cl.⁵: **A61K 35/16, C12N 5/00, C07K 3/02**

(30) Priority: **02.05.89 US 346124**

(43) Date of publication of application:
**07.11.90 Bulletin 90/45**

(84) Designated Contracting States:
**AT BE CH DE DK ES FR GB GR IT LI LU NL SE**

(71) Applicant: **ALLIANCE PHARMACEUTICAL CORPORATION**
**R.D. 1, P.O. Box 567, Sanatorium Avenue**
**Otisville, New York 10963(US)**

(72) Inventor: **Dinka, Stephen K.**
**R.D., No. 1, P.O. Box 537, Sanatorium Avenue**
**Otisville, New York 10963(US)**

(74) Representative: **West, Alan Harry et al**
**R.G.C. Jenkins & Co. 26 Caxton Street**
**London SW1H 0RJ(GB)**

(54) **Cell growth activators.**

(57) This invention relates to cell growth promoting materials or cell growth activators (CGA) which are present in human and animal blood (and other biological fluids). These cell growth promoting materials are preferably isolated or fractionated from serum or plasma via salt precipitation. The CGA can be used as an additive in nutrient media to promote the growth of tissues and cells therein as well as enhance or amplify the yield of proteins and other products.

EP 0 396 402 A2

# CELL GROWTH ACTIVATORS

This invention relates to cell growth promoting materials present in human and animal blood (and other biological fluids). There are described herein compositions and method for the preparation of such materials and the use of these materials for cell growth, cell proliferation, enhancement of cell growth, cell growth control, for substituting sera in cell cultures, for maintenance of cells, tissues, organs, and other biological material and to use in culturing fastidious microorganisms.

Moreover, the present invention relates to means for amplification of products cell-derived in cell cultures.

In one preferred embodiment, the inventive growth promoting materials are used as a media for growing cells as a replacement for, e.g., fetal bovine serum. Other uses, such as in vivo use to promote wound healing, are also envisioned.

Blood or serum is widely used as additive to culture media to grow wide ranges of fastidious microorganisms and cells which would grow only poorly or not at all in the synthetic medium only.

In general, blood or serum is used to provide these "growth factors." Blood or serum, however, is a very complex material containing many substances. Furthermore, the concentration of the "growth factors" in serum is very low, which makes their purification extremely difficult. See, S.K.Dinka: "Growth Regulation Factors in Serum Used For In Vitro Culture of Cells: Principles and Techniques; In: E. Kurstak; "Techniques in the Life Sciences, C̄1, Setting Up and Maintenance of Tissue and Cell Cultures" 1-33, (1985).

Scientists have sought means to isolate and purify substances responsible for growth. Some substances have been chemically identified and are polypeptides or proteins. Since such growth factors, however, are usually present in very low concentrations in blood, purification is extremely difficult. Techniques presently available for fractionation of growth factors from these complex systems generally are not appropriate for processing large volumes of serum (D. Gospodarowich and J.S. Moran, 45 Ann. Rev. Biochem. 531-588, 1976).

Methodologies available for the fractionation of proteinaceous growth factors from serum were reviewed in my earlier patents, as, e.g., U.S. Patent Nos. 4,189,535 and 4,346,816. The former patent teaches absorption of serum proteins to anionic exchange resins followed by specific elution of growth promoting materials utilizing gradient salt of pH solutions. The latter patent teaches the addition of perchloric acid to serum or plasma to obtain a precipitate, followed by extraction of growth factors by increasing the pH of the solution.

One group of growth factors in blood are the somatomedins, a group of circulating peptides presumed to be of liver origin that appear to be regulated by growth hormone. Somatomedins have been dissociated from larger carrier proteins in acidic environments, and it has been shown that active somatomedin molecules of less than 10,000 dalton molecular weight are present in soluble form in ultrafiltrates of human plasma after acidification (pH 2.3) with acetic acid (B.H. Ginberg, et al., 48(2) J. Cein Endocrinology and Metabolism 43-49, 1979). Somatomedins are in plasma protein precipitates formed during Cohn fractionation, and they are dissociated from the carrier proteins by acidification. The acid dissociation of somatomedins has been utilized in several methodologies for the collection of these growth promoters from Cohn-precipitated plasma proteins. (H. Burgi et al., 121 Biochem. Biophys. Acta. 349-359, 1966; Knut Uthne, 175 Acta Endocrinology (Suppl.) 1-35, 1973; E. Rinderknecht and R.E. Humble, 73-(7) Proc. Natl. Acad. Sci. U.S.A. 2365-2369, 1976).

During the blood coagulation process, growth factors also are released from blood platelets. Some researchers have claimed a major portion of serum growth promoters may be actually of platelet origin (see, e.g., R. Ross an A. Vogel, Cell 14: 203-210, 1978), As a result of the fact that growth factors account for only a small percentage of all serum protein, investigators have isolated platelet growth factors from preparations of lysed washed platelets utilizing ion exchange chromatographic procedures. C.H. Heldin et al. Exp. Cell Res. 109, 429-437, 1977, described two anionic fractions of 40,000 and less than 10,000 dalton molecular weight, respectively, and a heterogenous cationic fraction of from 25,000 to 35,000 dalton size possessing activity. Ross, et al. (in Hormones and Cell Culture, Book A, Eds. G.H. Sato and R. Ross, pp. 3-16 (Cold Spring Harbor Laboratory, 1979)), isolated a cationic fraction of 10,000 to 30,000 molecular weight. Further processing resolved mitogenic activity to molecular weight of 16,000 to 18,000 daltons.

These platelet-derived growth factors have been used to promote cell growth. However, these growth factors must still be mixed with platelet poor plasma in order to be effective.

Ammonium sulfate precipitation was used by Jacques and Barry (J. Gen. Physiol. 34:765-779, 1951) Eagle (Science, 122:43-46, 1955), Fisher et al. (Proc. Natl. Acad. Sci. U.S.A. 44:4-10, 1958), Liberman and Ove (J. Biol. Chem. 233:637-642, Michl. (Exp. Cell. Res. 23:324-334, 1961), Healy and Parker (J. Cell Biol. 30:539-553, 1966), Todaro et al. (Wistar Inst. Symp. Monograph 7:87-98, 1967), Paul et al. (Proc. Natl. Acad. Sci. U.S.A. 68:645-648, 1971), Lipton et al. (Exp. Cell Res. 74:466-470, 1972), Hoffmann (Exp. Cell Res. 85:275-280, 1973), Leffert (J. Cell Biol. 62-767-779, 1974), Slotta, et al. (Hoppe-Seyler's Z. Physiol. Chem. 356:367-376, 1975), McKeehan, et al. (Biochem. Biophys. Res. Comm. 80:1013-1021, 1978) used ammonium sulfate salt for separation of serum growth factors.

Most cell growth promoting activity was contained in the 40 to 50% ammonium sulfate concentration. Considerable activity resided also in fractions precipitated at other concentrations. John Bozicevich (U.S. Patent No. 3,429,867, Feb. 25, 1969) used ammonium sulfate for preparing serum substantially free from gamma globulin.

Drawbacks from the use of whole sera from animal sources are numerous. The most serious disadvantage is due to the fact hat spontaneous blood coagulation is time-consuming. Also due to the spontaneity of blood clotting, the time and completeness of clotting varies considerably with the donor source and the circumstances of collecting as well as processing and storing such blood.

It has been found that these factors have a decisive impact on the quality of the specific growth promoting activity of each serum batch.

Despite precautions taken in the isolation of whole sera, it has been found that the growth supporting efficacy is often jeopardized by the waiting period which may take a whole day or more and also by the somewhat difficult clot removal.

Depending on the individual animal and specific circumstances, the steps involved in the clarification of sera often result in certain uncontrolled biological processes such as proteolysis which may be deleterious to the specific activity of cell growth promoting or tissue culture-supportive factors per volume of sera.

The variability of this natural product thus has often distorted or diminished its utility in tissue cultures.

Another important drawback in the use of natural or spontaneous whole sera resides in the need to use many superfluous and perhaps undesirable serum components other than the desirable tissue and cell culture supporting factors.

One of the most commonly used natural products for growing cells is Fetal Bovine Serum (FBS). FBS is often in short supply since it is dependent on the fluctuation of the slaughter rates and beef consumption in this country. Accordingly, the price structure is fairly volatile and tends to be expensive.

Another consideration for potential hazards in the use of FBS or any other sera, for that matter, concerns the often undetected presence of contaminants such as, e.g., microbes or toxic chemicals. Among the microbial contaminants, one would include bacteria, viruses, plasmas and other pathogens. Among the toxic substances, but not restricted to, one would count such ingested products as hormones, insecticides, herbicides, heavy metals.

In view of the importance of the growth factors present in the sera of mammals and in recognition of the fact that the conventional procedure for obtaining the sera is slow and quite possibly hazardous to the specific activity of the sera as growth promoting additive to tissue culture media, the need for a reliable method for isolating the growth promoting fraction from blood in a safe, non-degradative, and rapid manner has become very important to the field of tissue culture.

It is the object of the invention, therefore, to provide a technique to isolate the growth-active fraction of blood without relying on the natural or spontaneous as well as highly variable process of blood coagulation in order to obtain blood or platelet free sera still capable of supporting in vitro cell growth.

It is another object of the present invention to provide a fraction of growth supporting material without significant loss of activity.

It is a further object of the present invention to provide fractions of growth activity by salt precipitation of plasma serum or defibrinated plasma with ammonium sulfate.

It is another object of the invention to provide growth promoting fractions by salt precipitation of plasma wherein the blood cells were first removed by centrifugation.

It is another object of the invention to provide growth promoting blood fractions of consistent and superior quality as compared to whole serum derived from spontaneously clotted blood volumes.

Another object of the present invention is the purification of human growth promoting salt fractions from blood, serum, plasma and other bodily fluids.

Another object of the invention is to avoid adding or at least diminish addition of undefined, unknown, and excessive amounts of serum proteins just to provide adequate support for growth to the synthetic tissue culture media.

Another object of the invention is to overcome the problems associated with the use of FBS such as described above.

It is still another object of the invention to provide growth promoting fractions from bodily fluids which enhance enriched production of proteins either accumulating intracellularly or secreted into the ambient growth medium.

The inventive growth factors derived from a plasma or plasma-derived serum fraction go a long way toward economical, safe and defined additive nutrient media.

A further object of this invention is the use of CGA preparation for the enhancement of tissue or organ culture as, e.g., in the in vitro culture of layers of collagen containing connective tissue. Such cultured quasi-organs have been found useful as replacement skin layers or temporary protective screens to help prevent water loss and offer protection against microbial infections, which is of critical importance to burn victims.

Fig. 1 represents a comparison of cell growth with fetal bovine serum and Biotis CGATM.

Fig 2 represents CHO cell growth (Fig. 2a) and product yield (Fig. 2b) in Medium I (Alpha modified DMEM) with 10% FBS or 3% CGA-3 + E.

Fig. 3 represents CHO cell growth (Fig. 3a) and product yield (Fig. 3b) in Medium II (Williams medium E) with 10% FBS or 3% CGA-3 + E.

Fig. 4 represents fibroblast cell growth (Fig. 4a) and product yield (Fig. 4b) in Medium I with 10% FBS or 3% CGA-3 + E.

Fig. 5 represents fibroblast cell growth (Fig. 5a) and product yield (Fig. 5b) in Medium II with 10% FBS or 3% CGA-3 + E.

Fig. 6 represents B hybridoma cell growth (Fig. 6a) and product yield (Fig. 6b) in Medium I with 10% FBS or 3% CGA-3 + E.

Fig. 7 representsB hybridomacell growth (Fig. 7a) and product yield (Fig. 7b) in Medium II with 10% FBS or 3% CGA + E.

Fig. 8 represents T hybridoma cell growth (Figs. 8aa and 8b) in Medium II with 10% FBS or 3% CGA-3 + E.

Fig. 9 represents the effect of MCDB 302, Alpha MEM and Iscove's Modified DMEM media on the growth of CHO-FCR cells.

Fig. 10 represents the effect of MCDB 302, Alpha MEM and Iscove's Modified DMEM media on the growth of CHO plant cells.

Fig. 11 represents the effect of MCDB 302, Alpha MEM and Iscove's Modified DMEM media on the growth of CHO-K1.

Fig. 12 represents Best Medium and CGA for CHO-K1 cells.

Fig. 13 represents the effect of MCDB 302 Apha MEM and Iscove's Modified DMEM media on the growth of CHO-FCR cells.

Fig. 14 represents CHO-K1 cell growth with CGA BC 2000 in suspension culture.

Fig. 15 represents the effect of Cell growth Activators (CGA's) and Fetal Bovine Serum on the product yield of CHO-H$_1$ cells.

Fig. 16 represents Best Medium and CGA for chick embryo cells.

Fig. 17 represents chick embryo cell growth in roller tubes with 5% FBS or CGA-3.

Fig. 18 represents the effect of CGA's on chick embryo cell growth and SB-1 virus yield.

Fig. 19 represents the effect of CGAS on chick embryo cell growth and HVT virus yield.

Fig. 20 represents the effect of CGA's on MDBK cell growth and BVD virus yield.

Fig. 21 represents the effect of IBR virus titres in MDBK cells grown in CGA-2 and CGA-3.

Fig. 22 represents the mean product yield enhancement for 13 lots of CGA-3 with CHO-FCR cells passaged in 10% FBS or 1.5% CGA-3 + E.

Cell growth promoters as serum substitutes are isolated from plasma, defibrinated plasma, plasma-derived serum, and serum (derived from blood after removal of clot).

The purification of water-soluble proteinaceous growth activators is conveniently accomplished by selective precipitation of growth promoting fractions. As is well-known in the art, ammonium sulfate salt can be used to precipitate various kinds of proteins depending on their size and overall charge. This method has been very much in use for the isolation of blood albumin, immunoglobulins and other such components.

With step-wise increases of the ammonium sulfate concentration in serum or plasma, another distinct population of proteins is precipitated. After each step, the precipitated material can be separated from the solution by the conventional gravitational techniques such as by centrifugation or filtration.

The process of isolation is described in two preferred methods. The first method entails a one-step precipitation with ammonium sulfate which is added in solid or liquid form to the clarified cell free residual blood fluid such as plasma or serum of the different kinds mentioned above. The final concentration of ammonium sulfate at about room temperature is brought up to 30% saturation.

In the second embodiment of the claimed invention, a multiple step precipitation method is employed according to the following general protocol.

The purified blood fluid volume, e.g., plasma as described, is mixed with ammonium sulfate in either solid or liquid form. The incrementally attained concentrations of ammonium sulfate are about 20%, 30% 40%, 50%, 60% and 70% (w/v) saturation at about room temperature or a designated temperature, e.g., 4° C.

At each incremental step of ammonium sulfate salt concentration, the precipitates are separated or collected by conventional means such as, e.g., centrifugation. The precipitated and pelleted material is re-suspended and dissolved in one-tenth the reaction volume or in any desired volume of physiological saline solution, containing about 0.85% sodium chloride.

Each re-dissolved fraction is dialyzed against the same physiological saline solution, at an adjusted pH range from about 7.2 to about 7.6.

After the extraction of the various incremental fractions of growth factors has been thus completely resolubilized by dialysis, the osmolarity of each fraction is determined and, if necessary appropriately adjusted with saline before filtration through sterile 0.22 micron pore size membrane filters. Thereafter, the fractions can be stored frozen, preferably at or below about -20 degrees C.

The blood plasma or serum supernatant fractions of the precipitation and separation steps are mixed with a sufficient amount of ammonium sulfate (in either liquid or solid form) in order to increase the salt concentration to the next appropriate or suitable increment. These precipitations and separation steps are repeated at each increment and terminated with the final step which effects precipitation at about 70% (w/v) saturation with ammonium sulfate. Centrifugation and separation is again followed by dialysis of the supernatant against physiological saline solution, as described. Optionally, the 70% (w/v) precipitation step can be omitted in favor of using the supernatant after removal of the 50% saturated ammonium sulfate precipitated protein.

The 50%, 60%, or 70% (w/v) ammonium sulfate precipitated material is either re-dissolved as described above in solutions in the about 20% to about 35% or preferably 25% to 30% (w/v) saturated ammonium sulfate range or, directly, in about 1/10th of the starting volume or in a desired volume of saline solution and then dialyzed.

Alternatively, a still quicker process provides for mixing the supernatants at each step without the dialysis interval directly with a sufficient amount of ammonium sulfate solution in order to attain the next increment of precipitation of blood plasma or serum protein. However, the growth-stimulating activity remaining in 50% saturated ammonium sulfate supernatant serum or plasma has been found efficacious in enriching several media (see Examples).

The step-wise precipitated proteins generally vary according to their biological and physicochemical properties. Each fraction is analyzed as to its growth-enhancing properties. It has been discovered that a cut of proteins derived from 30% to 50% saturated ammonium sulfate contains a composition with most of the growth supporting activity of the unprocessed plasma or serum. At the same time, this procedure avoids the variables contributed by a degree of incomplete blood clotting which often leads to activation of proteolysis.

Another benefit accrues from the fact that the blood cells are more likely to stay intact during centrifugation as compared with the situation created by spontaneous clotting. Moreover, not all cells may be removed by clotting which is frequently incomplete. Hemolysis and its concomitant release of cellular components into the plasma or serum may play a role in the variability of growth enhancing capacity.

The following example is to illustrate the effectiveness of the blood fractions as practiced according to the present inventive isolation technique. Aside from avoiding the laborious and wasteful removal of the blood cells by clotting, the growth-enhancing properties of the composition are often found to be as effective as, or better than, the conventional serum product.

The salt precipitation, of course, is not limited to ammonium sulfate but includes other suitable, physiologically amenable salts. These salts include, but are not limited to, potassium sulfate, sodium sulfate, magnesium sulfate, potassium chloride, and sodium chloride.

The various fractions derived from plasma or serum can be tested for tissue or cell supporting activity by the known criteria of cell yield, cell doubling time, $[^3H]$-TdR uptake, increased proteins, and specific enzymatic or biological assays. In addition, the composition of the fractions can be assayed for the presence of proteins and contaminants such as, hemoglobin and other plasma or serum components as well as blood cell lysis products.

Routinely, the fractions are filtered through sterile filter membranes having holes of about 0.2 microns, or, alternatively, about 0.45 microns diameter.

For special purpose uses, the fractions can be tested for the presence of viruses or mycoplasmas by the appropriate known methods as applied in the case of conventional whole serum or plasma.

For use as an additive in the tissue culture media, the stock solution volume of the purified fraction is conveniently held nearly the same as the starting volume of plasma or serum if not concentrated. Therefore, plasma or serum fractions enriched in growth promoting substances can be used in the media at volumes equivalent to the factor of concentration is considered for the addition of adequate amounts of growth factor to the media same percent (v/v) concentrations as the conventional sera. Consequently, compositions of tissue culture media can contain the growth promoting solutions at suitable volume concentrations usually varying from 1% to 6% (see Examples).

The purified fractions isolated from plasma or serum have been tested for their cell growth stimulating activity in cell proliferation tests as compared to the conventional untreated materials.

Another advantage of the invention can be found in the fact that some cell lines respond particularly and selectively well to the 10% to 30% salt ammonium sulfate precipitated fractions as compared to the fractions in the 30% to 50% range.

The process for isolation of the growth supporting or promoting fractions of plasma or serum can be, of course, applied to the blood or other bodily fluids harvested from about any suitable animal source. Thus, in addition to beef, other suitable sources can be swine, goat, sheep, horse, rabbit, duck, turkey, chicken, etc.

Furthermore, the growth promoting activities of plasma or serum fractions can be lyophilized for long term storage or easy transport.

Cell Growth Activators (CGA's) are native proteins, which are present in the blood effecting and controlling the growth and division of cells, and are obtained through the novel proprietary process(es) as described herein. I have found that these factors also effect the protein expression, the secreted or accumulated product yield, as the amount of pharmaceuticals or viruses produced by these cells. However, the nature or the mechanism of biological action of these native circulatory proteins is not yet understood. There is no direct evidence as to how such proteins influence protein expression or yield of secreted protein. It has been generally assumed that CGA effected protein expression parallels the cell growth and division as the cell goes through the cell cycle. This assumption may no longer be true.

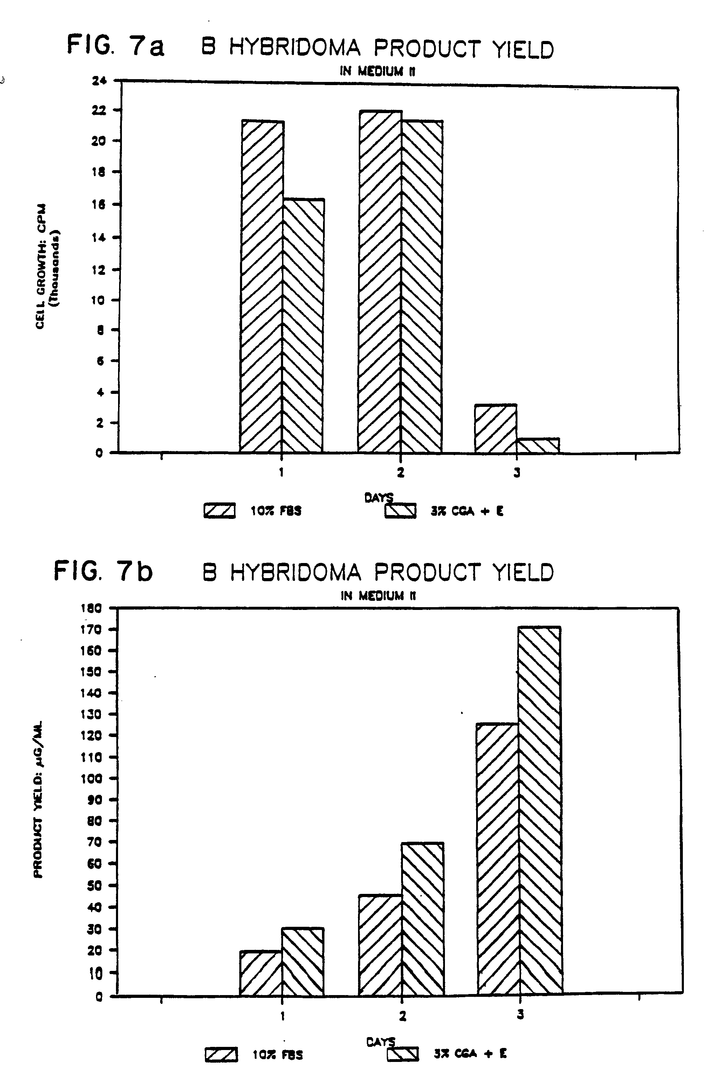

Since the individual CGA's are not characterized, their individual concentrations in the blood cannot be measured. Their apparent activity, therefore, was tested in serum fractions only (undefibrinated plasma would clot in cell culture media) by measuring cell growth using established parameters. This method, however, does not reveal any information about the individual components or their relative proportions. A further complication derives from the fact that the activities are not necessarily additive or synergic. To varying extent, they can both enhance or inhibit each other depending on concentration. An concentration beyond a certain optimum range can even cause dose dependant inhibition (See Fig. 7). The presence of a single factor or a plurality of factors having no activity by themselves can, however, drastically increase the cell growth promoting activity when combined with active proteinaceous compounds.

A preferred embodiment of the present invention is demonstrated, but not in any way limited, by the following Examples.

In these Examples, the inventive compositions are represented by their "CGA" numbers or their "BC" numbers. Here, CGA-1 is BC 1000, derived from the 30% precipitate. CGA-2 is also known as BC 2000, which is derived from the 50% precipitate. CGA-3 is BC 3000, which is from the 50% precipitation step supernatant.

These Examples and figures also refer to a cell growth Enhancer E. Enhancer E is derived from lysed red blood cells. It has little or no cell growth promotion activity of its own, but when mixed with water CGA preparations, it enhances cell growth significantly. As shown in the preferred embodiment (Examples), Enhancer E is usually added at 1% (v/v) concentration.

Enhancer E is disclosed and claimed in copending U.S. Patent Application Serial No. 131,188, filed December 10, 1987. That application is incorporated herein by reference.

Example 1 CGA Preparation

For the production CGA's, it is preferred to use instead of whole blood either blood plasma or serum. The plasma which is produced in the presence of Na citrate anticoagulant is treated by further removal of the blood cells and platelets in order to be used in production of CGA's. Serum is obtained by allowing the whole blood, containing the blood cells and platelets to clot. During clotting it undergoes a very complex proteolytic cascade reaction. Through this process proteins are effected by activated proteolytic enzymes and the cells and platelets release additional cell growth promoting proteins and other substances. This activities are affected by age, sex, season, feed, environmental as well as stress related and other conditions. The serum as such can be considered a pathological fluid. Conversely, the plasma reflects more closely the sum of normal cell growth promoting activators, of a living organism.

Example 2

The product, the CGA Preparation, was aimed to provide good, cell growth comparable to fetal bovine serum for specific cells.

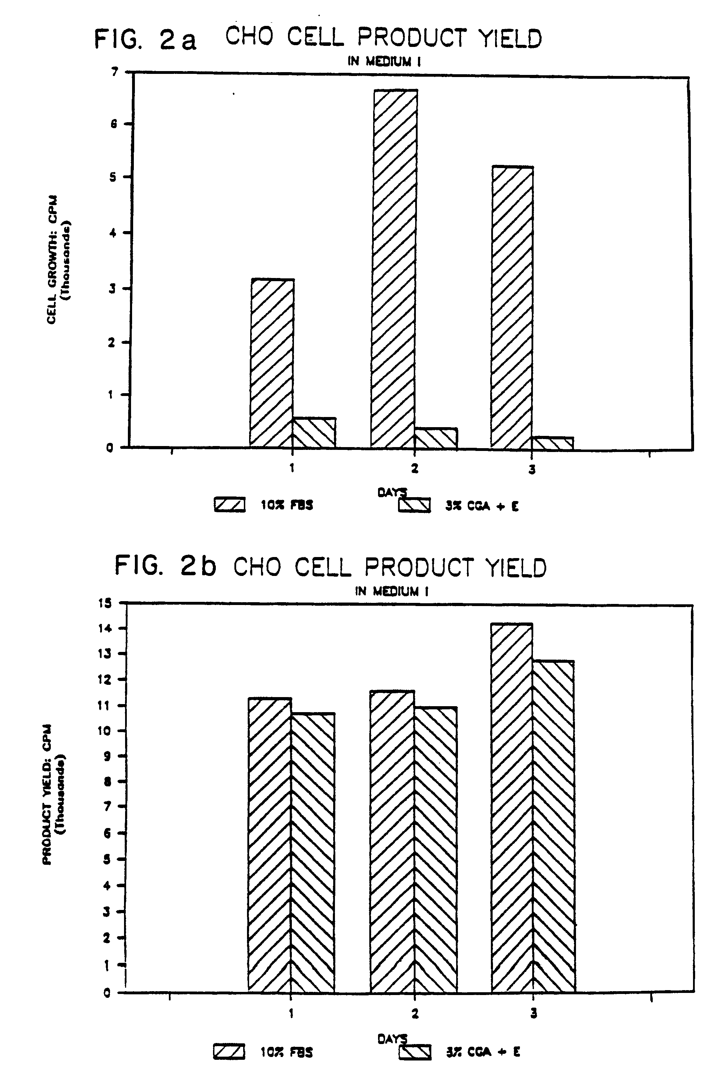

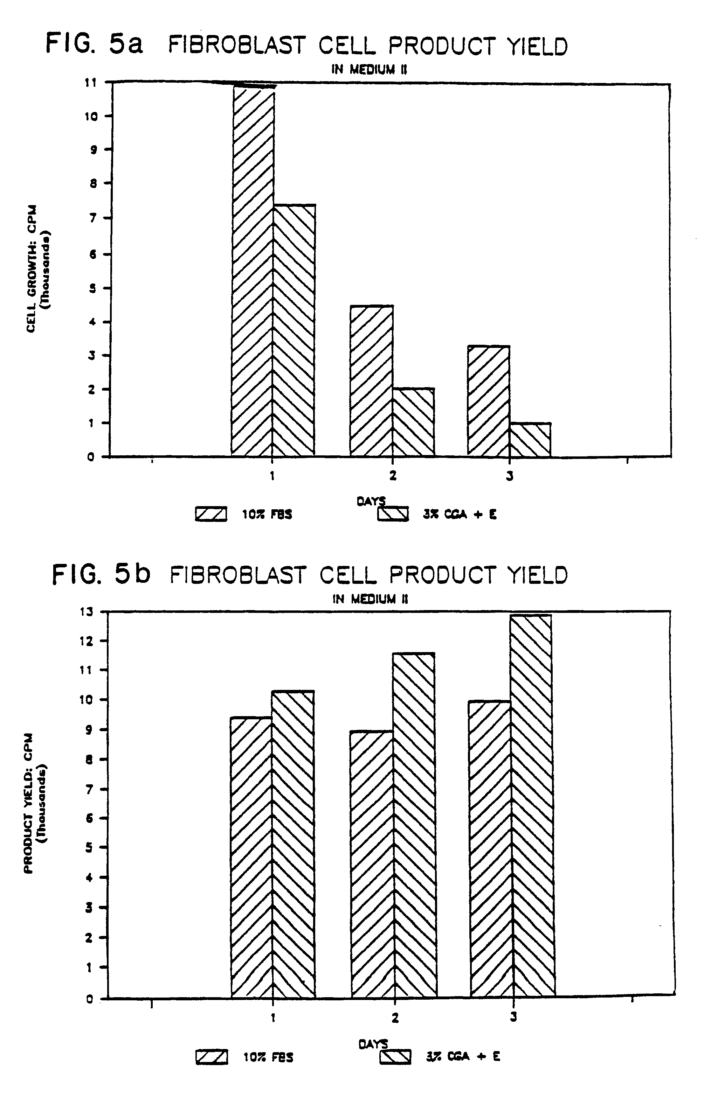

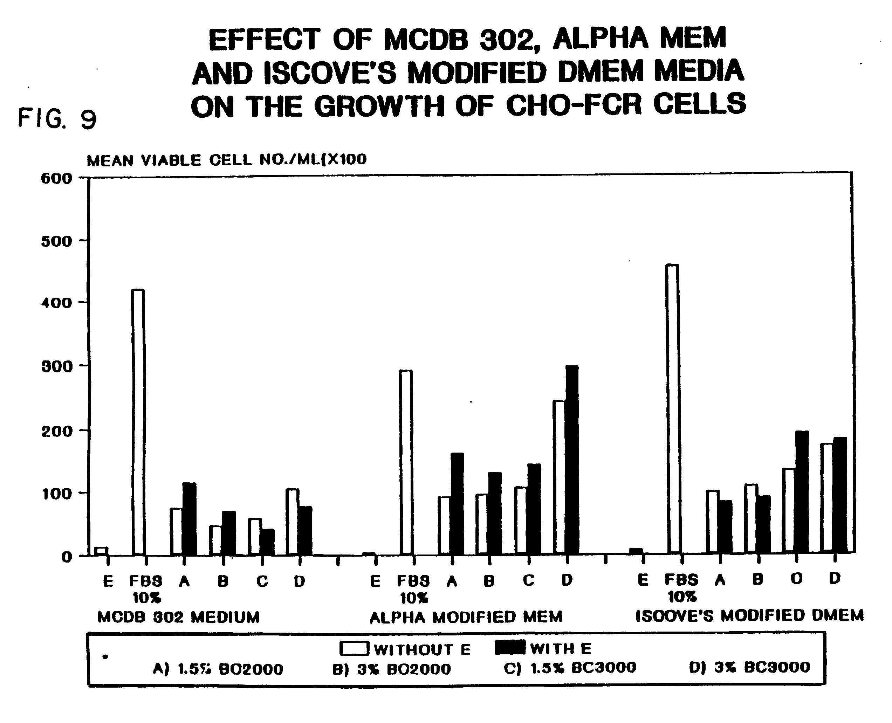

The effects of CGA fractions in two different recombinant cell lines (one hybridoma and one T cell line) unexpectedly showed that the CGA-rate of cell growth promotion differed from the CGA rate of activation of protein expression (Fig. 2, 3, 4, 5, 6, 7, 8). Subsequently, I also found with other cells that the CGA·fractions exerted different effects on the cell growth rate, protein expression and also cellular product yields. The surprising results varied not only from one cell type to another but also among cloned cultures which derived directly from the same single parental cell, as would be the case with hybridoma or recombinant cell lines. (Fig. 9, 10, 11).

Example 3

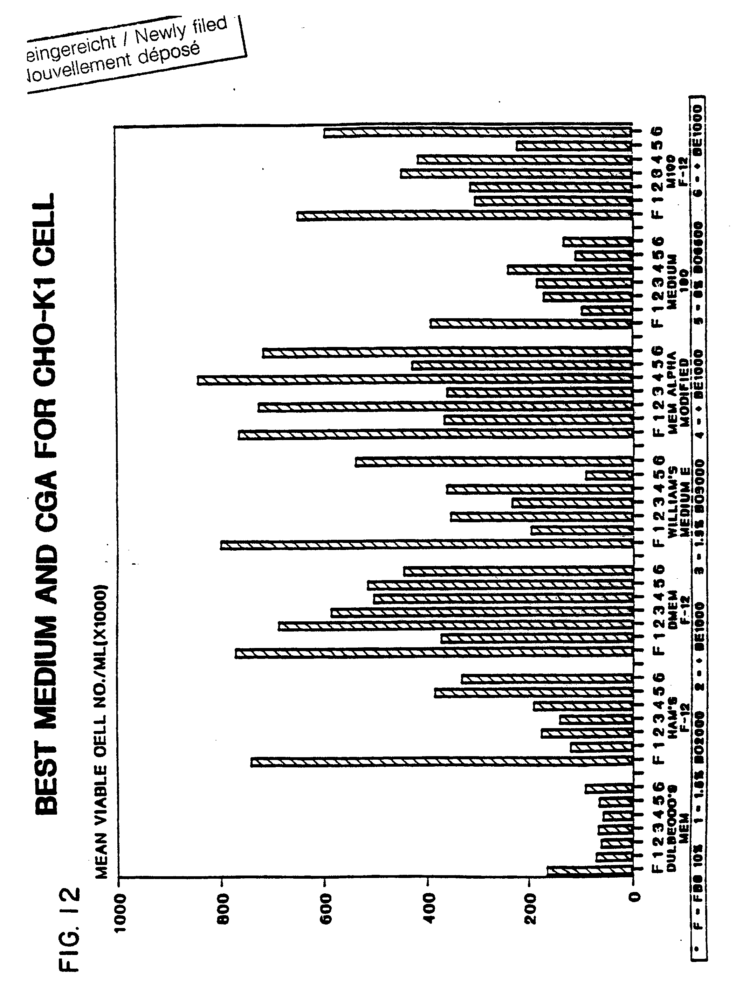

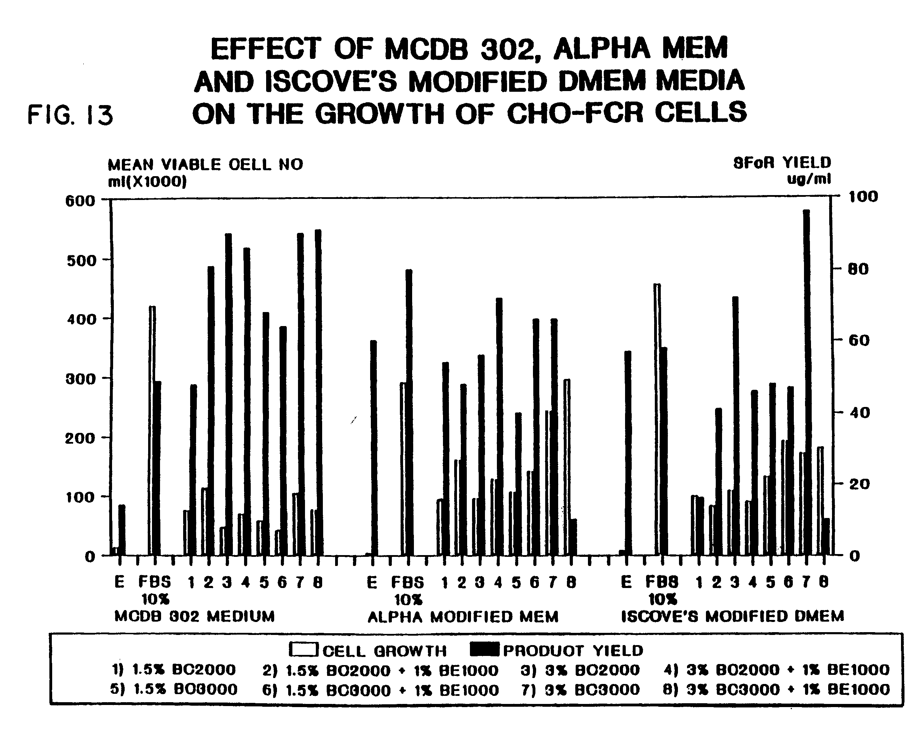

Even in the virtual absence of cell growth, protein expression and secretion rates due to the CGA fractions were found to be as high as or higher than in the presence of fetal bovine sera. The cell growth rate therefore can not be taken as reliable indicator for product yield as well as the rate of protein expression as I previously assumed. Moreover, I discovered that the specific composition of Basal Medium has an apparently critical contributory effect in conjunction with the CGA fractions (Fig. 12, 13).

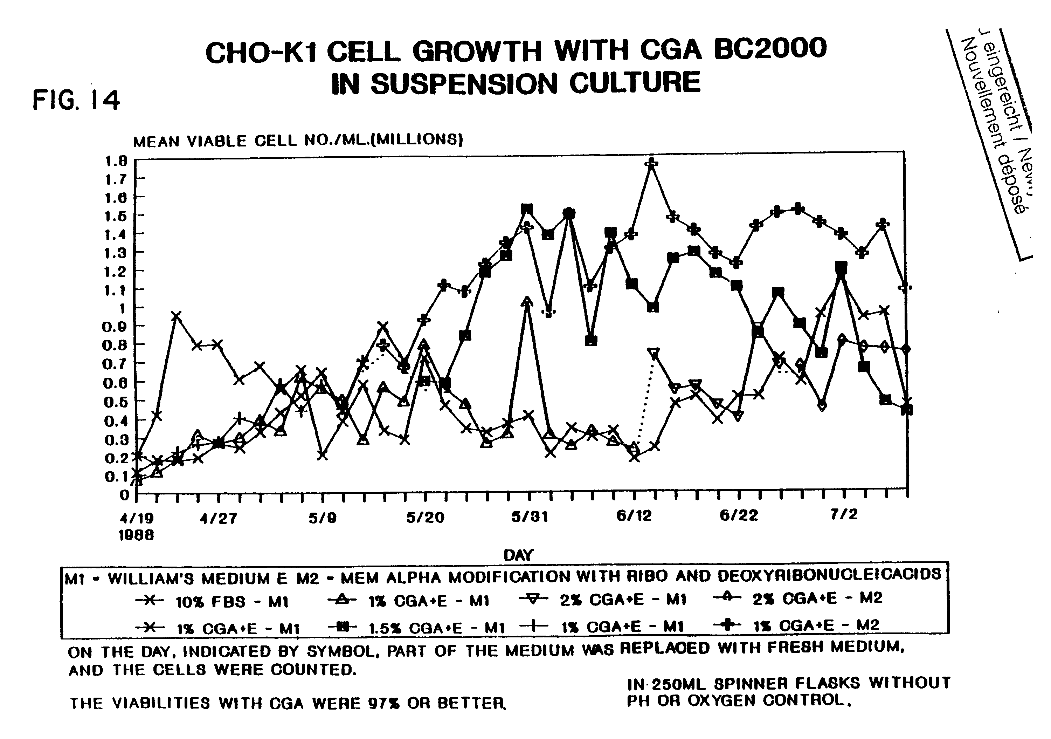

Indeed, the preferred combination of Basal Medium and CGA for optimum cell growth may be surprisingly different from the combination which is effective for the optimum product yield, or protein expression (Fig. 13, 14).

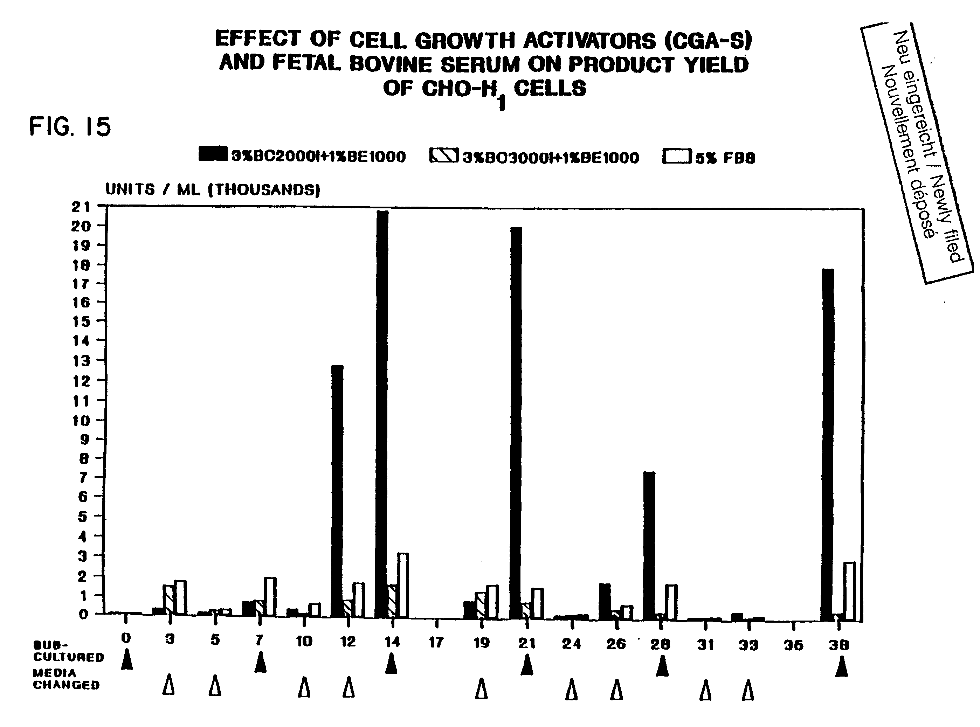

The present preferred embodiments include combinations which vary in effect not only from one cell type to another but even for cells derived from the same parental cells, like hybridomas and recombinant cells. One set of data shows the product yield from CGA treatment to be up to 10 times higher than with fetal bovine serum (Fig. 15).

Example 4

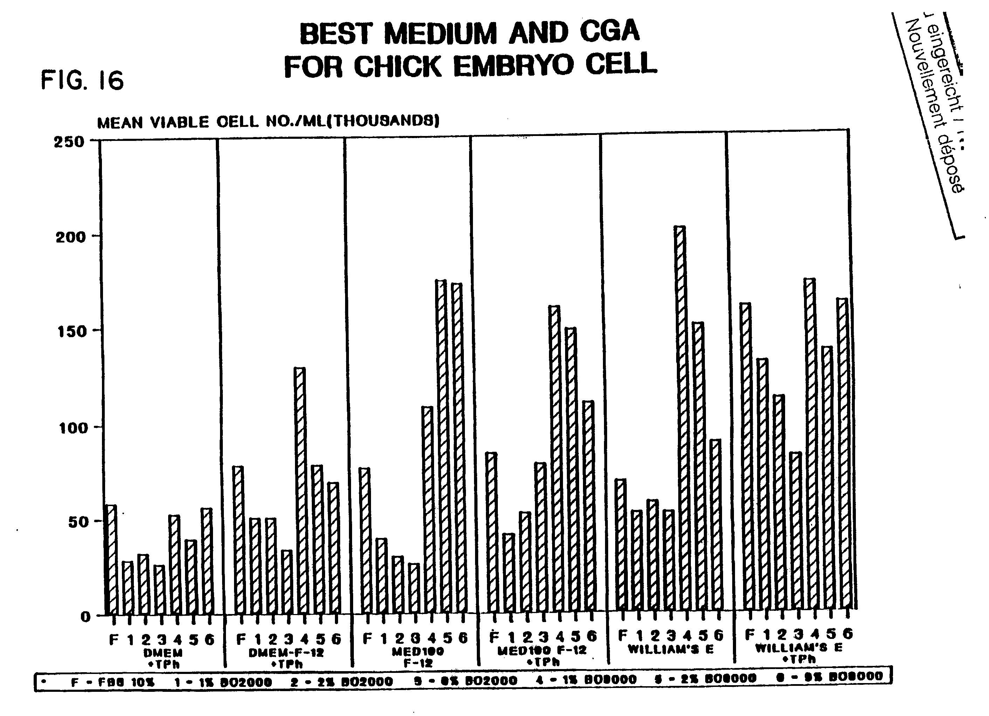

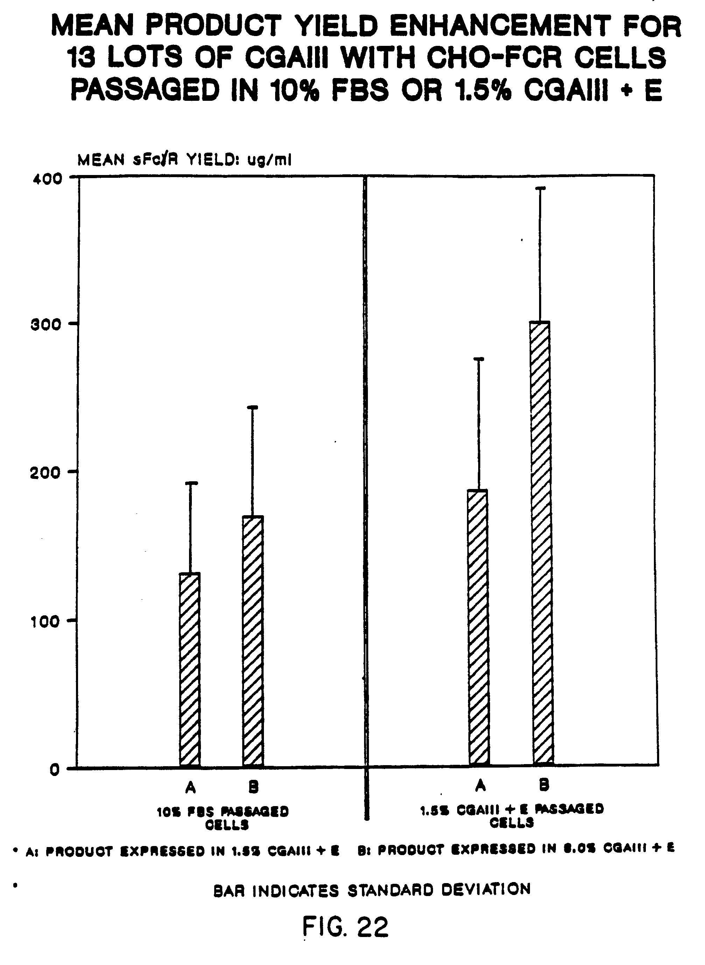

Our experimental data indicate that CGA a has similar effect on virus replication in cells which are used for virus vaccine production (Fig. 16, 17, 18, 19, 20, 21). The virus yield that may turn out to be higher with low cell growth than with fetal bovine serum at high cell growth. Our recent data indicate that cells serially subcultured with CGA can have several times higher product yield, protein expression than cells grown with fetal bovine serum (Fig.22).

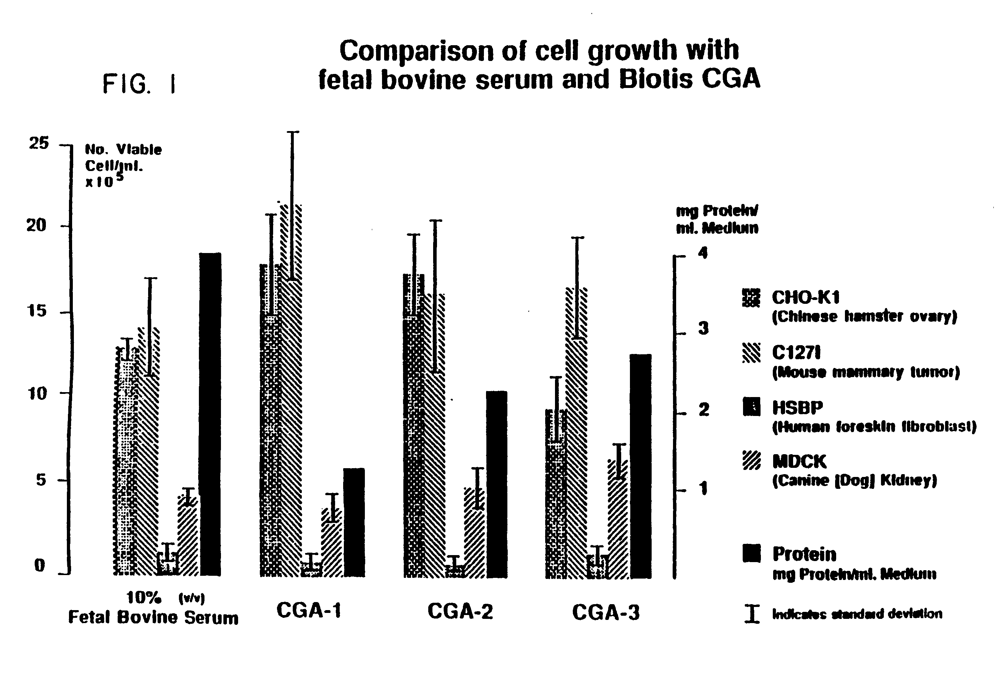

Example 5 (Fig. 1)

Cell growth was assessed in the presence of either 10% (v/v) FBS or 1%, 2%, and 3% (v/v) CGA fractions (CGA-1,-2, and -3, respectively (Biotis CGATM). Specifically, CHO-K1 cells (Chinese hamster ovary); C1271 cells (mouse mammary tumor), HSBP cells (human foreskin fibroblast), and MDCK cells (canine kidney) were grown in BME cultures. I discovered that the CGA fraction all exhibited excellent growth promoting effects by the additives CGA-2 and CGA-3 than CGA-1. CGA-1, on the other hand, effected greater growth rate than CGA-2, 3, or even FBS.

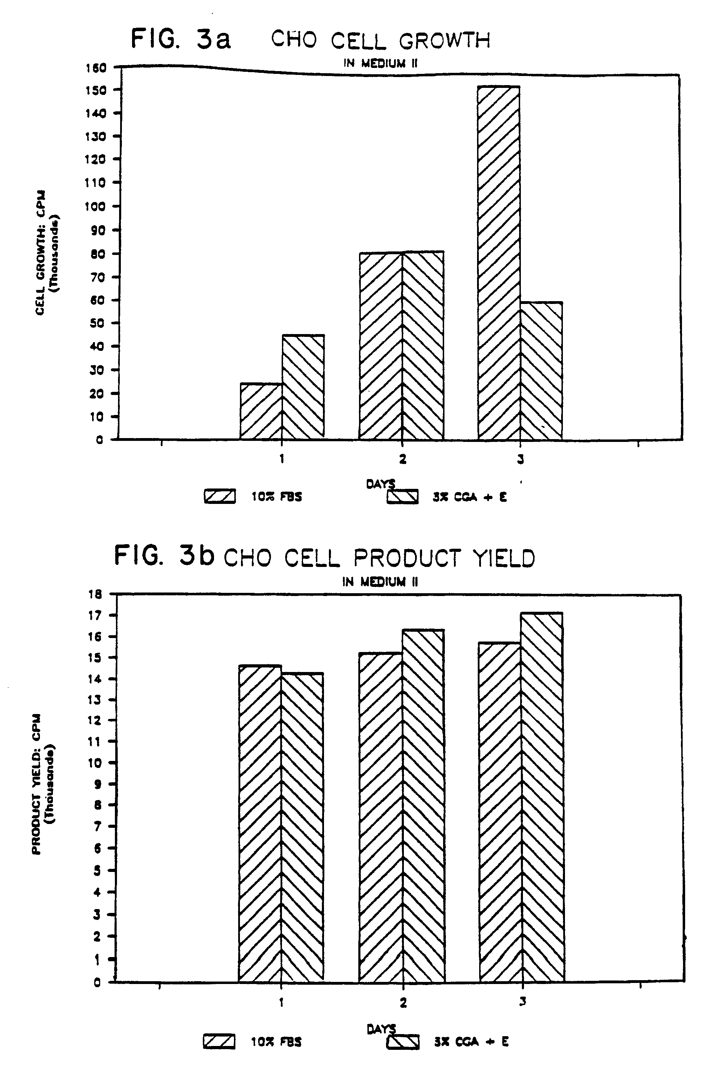

Example 6 (Figs. 2 and 3)

CHO cells were cultured in the presence of either 10% FBS or 3% CGA plus E (E is the hemolysis product as described in the U.S. Patent Application S.N. 131,188, currently pending). The additive E is always used in the overall concentration of about 1% (v/v). The results shown in Fig. 2 and 3 demonstrate that the cell growth rates (cpm incorporated) are sharply reduced in media containing CGA-3 (plus E) when compared to CHO-cultures with 10% FBS. However, the product yield, as determined by the counts incorporated in cellular protein secreted into the media, is about equal to the yield effected by FBS.

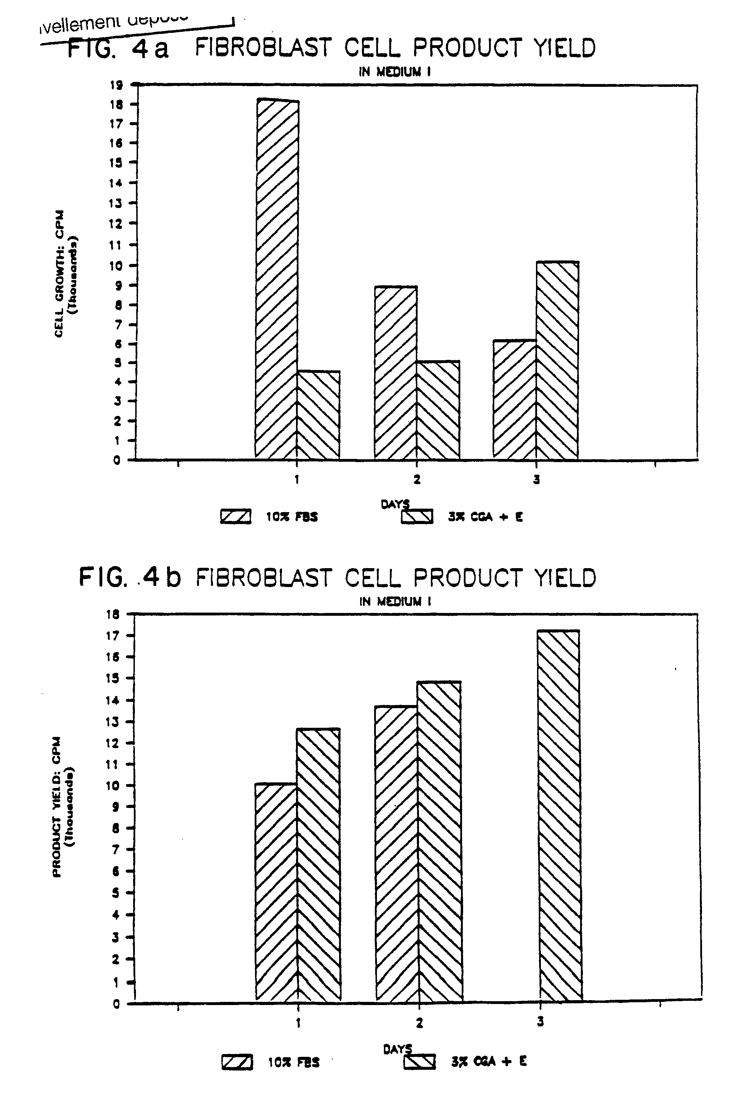

Example 7 (Fig. 4)

Cultures of HSBP fibroblasts, exhibited effects on growth and productivity similar to the CHO cell cultures.

Thus, the product yield effected by CGA-3 over a period of 3 days showed increasing secretion. Conversely, the growth rate was relatively slow. The 10% FBS containing cultures exhibit a sharp uptake of precursor on the first day which rapidly diminished on the following days.There are no data for the FBS effect on the product yield of fibroblasts after 3 days.

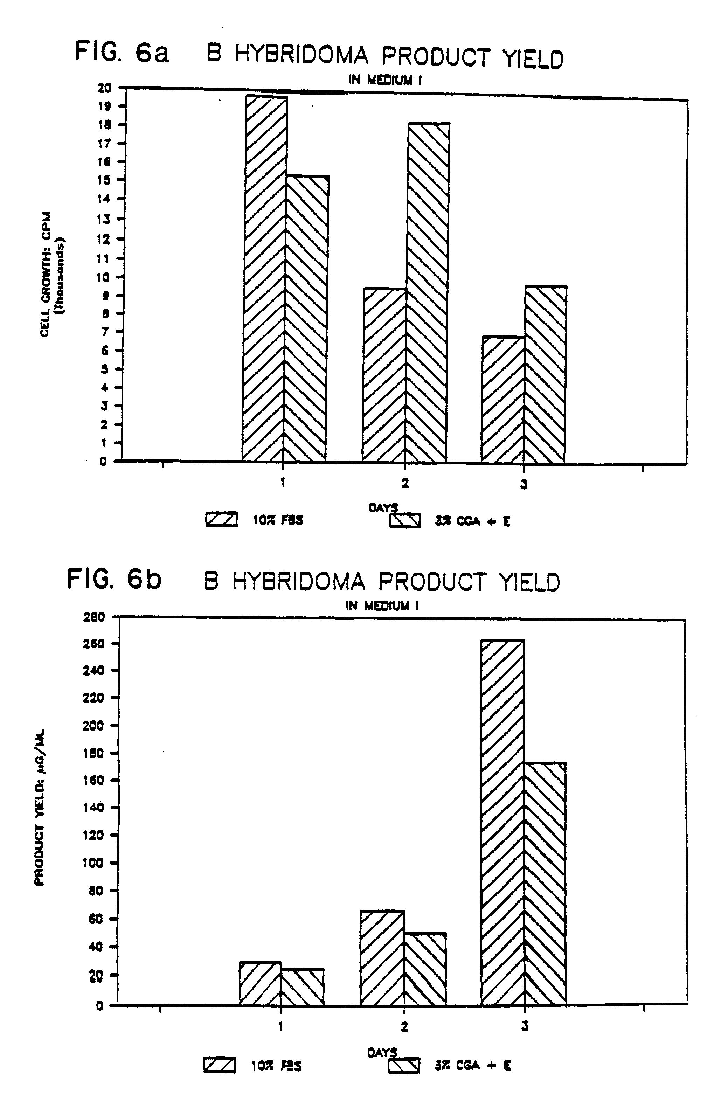

Example 8 (Figs. 6 and 7)

B Hybridoma cell growth was compared over 3 days with either 10% (v/v) FBS or 3% CGA + E (E, as described). While cell growth of hydridoma slowed down dramatically on the 3rd day, the product secretion continued to increase.

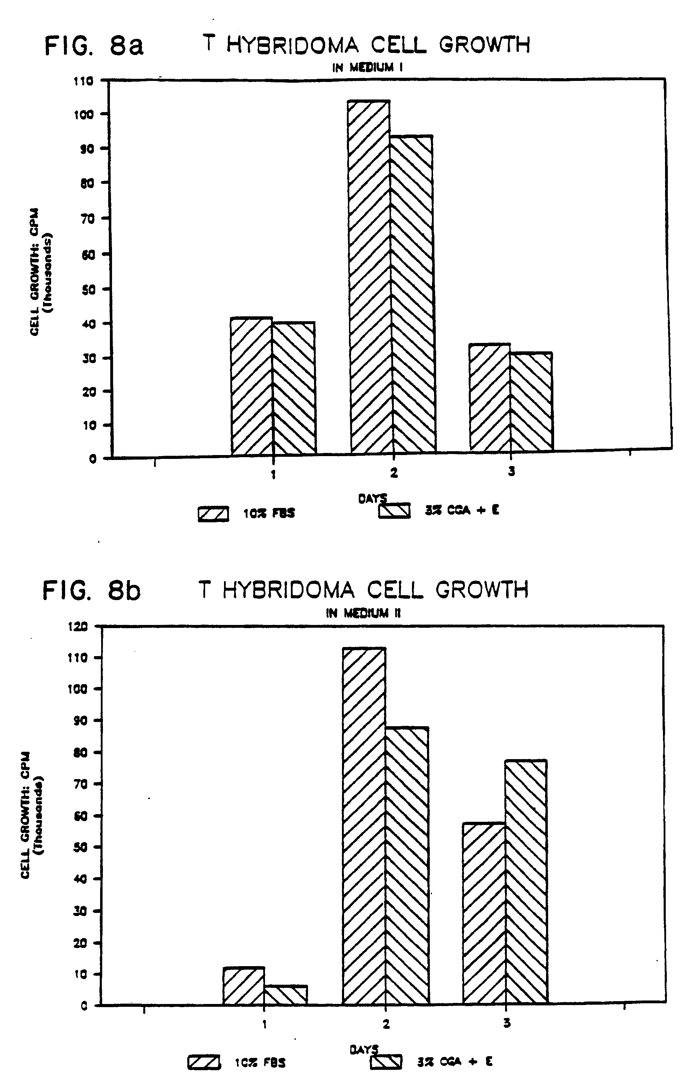

Example 9 (Fig. 8)

This experiment demonstrates that the T-cell hybridoma culture growth rate was maintained more vigorously in Medium II than Medium I, regardless of additive, FBS or CGA.

Example 10 (Fig. 9)

In a comparison of the effect of MCDB 302, ALPHA MEM, and ISCOVE'S Modified DMEM Media on the growth of CHO-FCR Cells, the media were combined with 10% FBS, 1.5% BC2000, 3% BC2000, 1.5% BC3000, or 3% BC3000 plus or minus E. Clearly, the Alpha modified MEM with 3% BC3000 exhibited the best growth promoting activity albeit not as high as FBS.

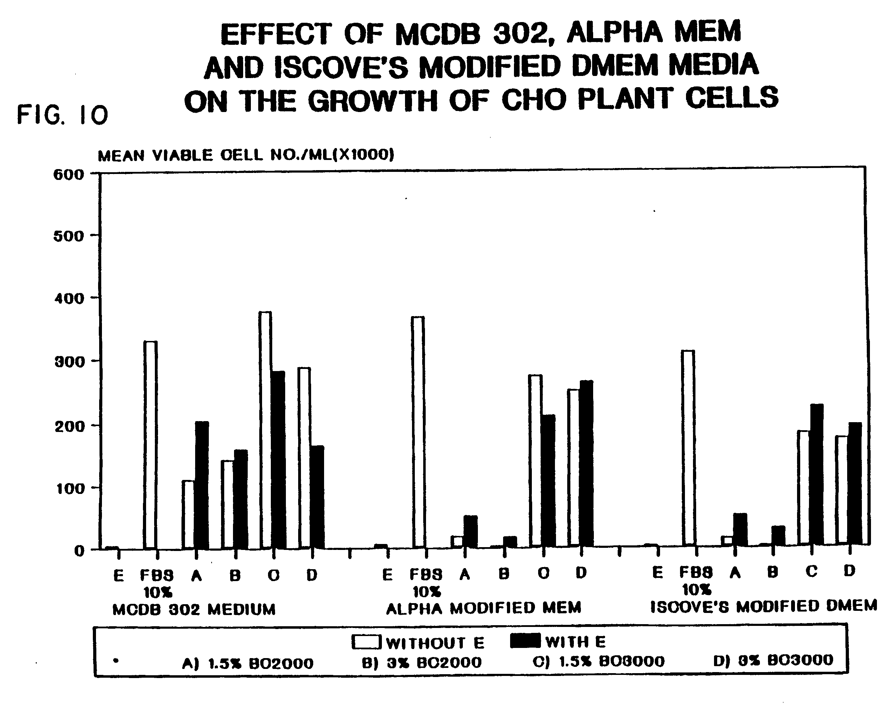

## Example 11 (Fig. 10)

The growth of CHO-Plant recombinant cells was comparatively enhanced in the preferred culture media of MCDB 302, Alpha modified MEM, and ISCOVE's Modified DMEM when grown in the presence of 1.5% and 3% BC3000. The addition of E also showed a promoting effect in the MCDB 302 in combination with 1.5% BC2000.

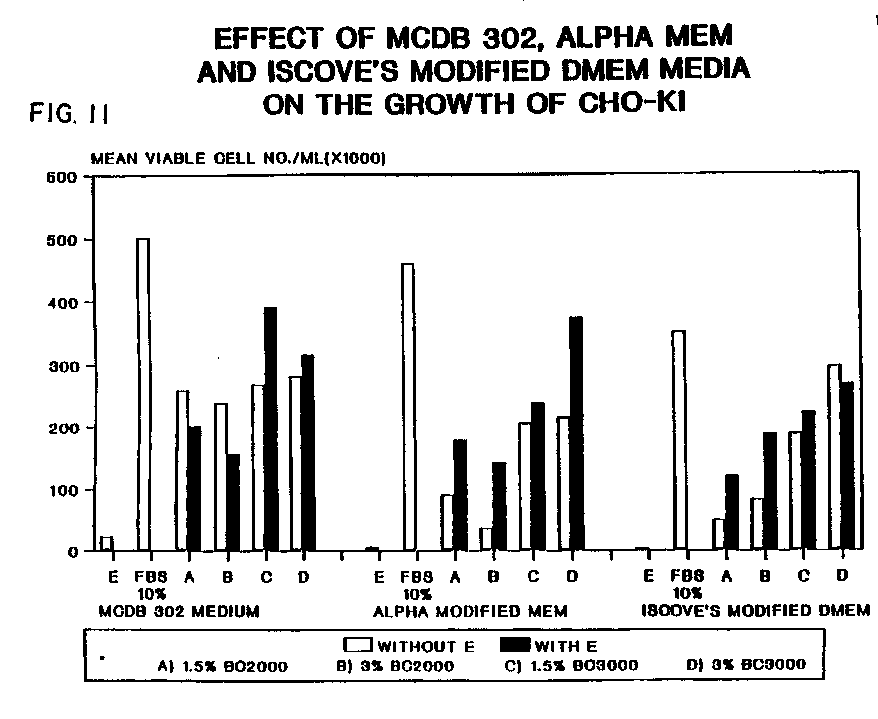

## Example 12 (Fig. 11)

CHO-K1 cells appeared to grow more vigorously in MCDB 302, regardless of growth factor, while ISCOVE's Modified MEM enriched with 3% BC3000 showed the greatest growth promoting effect.

## Example 13 (Fig. 12)

Tests for the mean viability on CHO-K1 cells in various media revealed DMEM F-12 plus 1.5% BC 2000 plus BE1000 (1% E) and Alpha modified MEM plus BE1000 (1% E) as most effective.

## Example 14 (Fig. 13)

The product yield appeared particularly enhanced by the growth factors of the present invention in those media which did not support a fast growth rate.

When comparing the product yield (SFCR in mg/ml) from CHO-FCR cells with the cell number a particularly good ratio is found in cells grown in MCDB 302 medium plus any combination of CGA plus or minus E.

## Example 15 (Fig. 14)

CHO-K1 cell long term viability in suspension cultures was shown to be outstanding when either 1.5% CGA + 1% E-M1 (William's E) or 1% CGA-2 +1% E-M2 (Alpha medium), where E is as described above, added to William's medium. Overall, the viability of CHO-K1 grown cells in spinner flasks was 97% or better in 2 1/2 month long cultures.

## Example 16 (Fig. 15)

Tests show that CHO-H$_1$, cells grown in the presence of CGA-3% BC2000 + 1% BE1000 outproduce cells grown 5% FBS or 3% BC3000 + 1% BE1000 five to ten fold.

## Example 17 (Fig. 16)

The data of Fig. 16 show a comparative series of chick embryo cell cultures exploring the best combination of medium and CGA. It is evident that Medium 199 F12 with added 1% BC3000, 2% BC3000, or 3% BC3000 are significantly more growth stimulating than the same medium with 10% FBS. Especially growth-effective was also the William's E medium plus 1% BC3000.

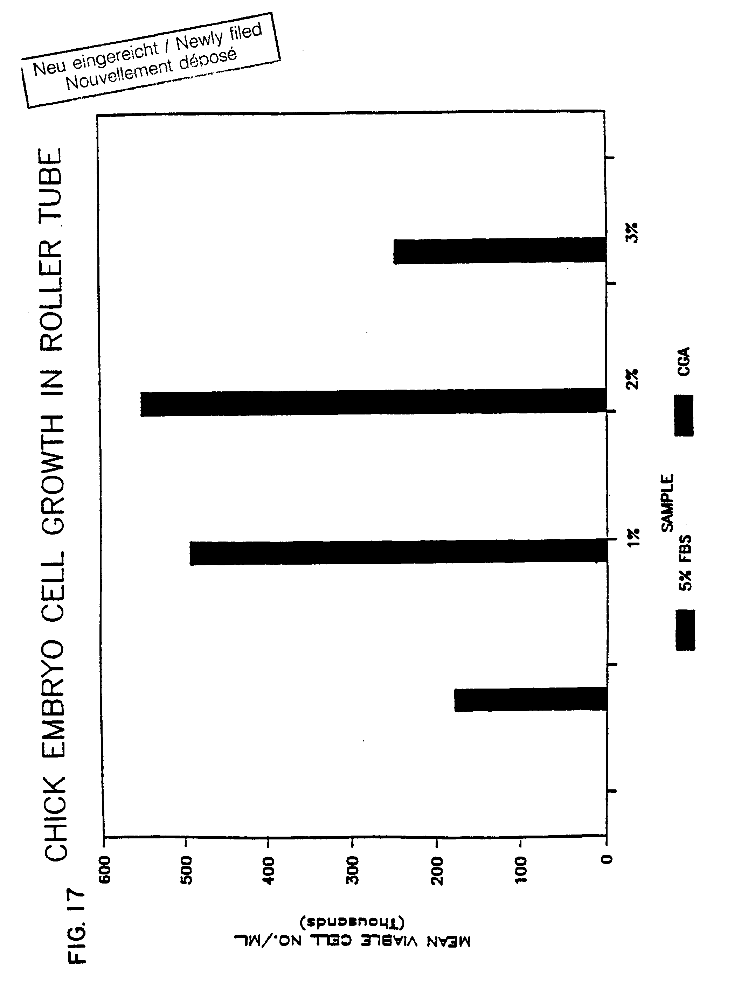

## Example 18 (Fig. 17)

The concentration effect of CGA on chick embryo cell growth in roller tubes is demonstrated to be more than doubling the rate as against 5% FBS when using 1% or 2% CGA.

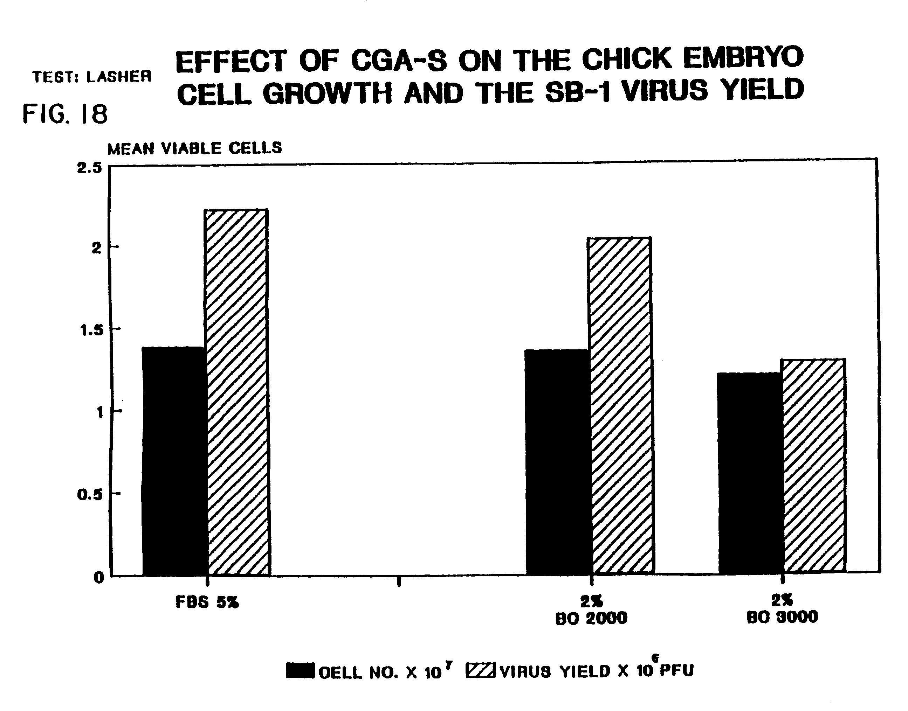

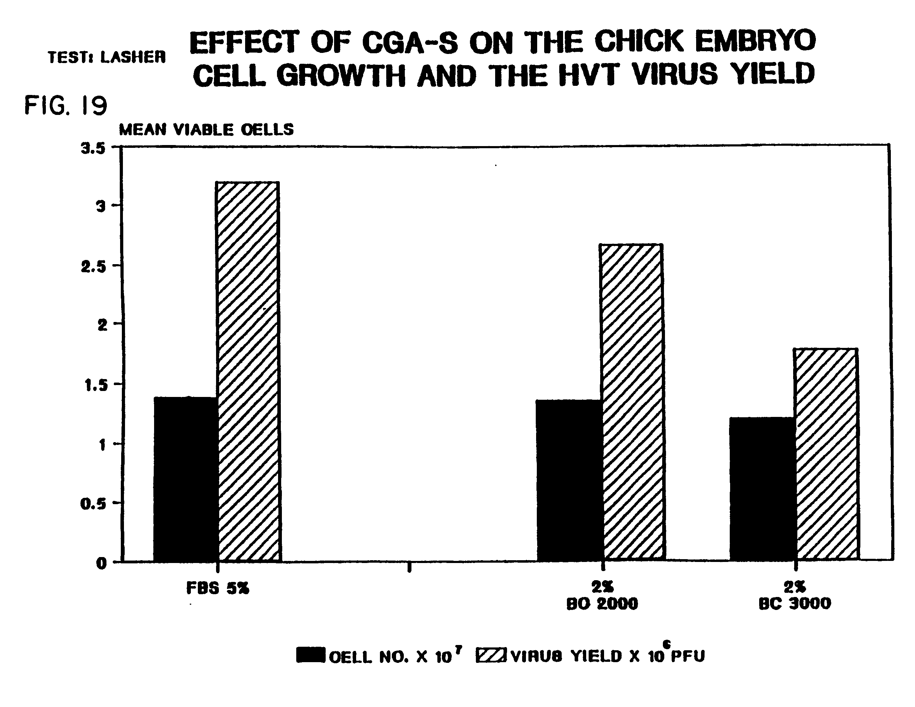

## Example 19 (Figs. 18 and 19)

Using the preferred 2% CGA (BC2000) enriched media to grow chick embryo cells infected with SB-1 virus or HTV virus, both cell growth and virus product were observed to be about equal to 5% FBS-containing media.

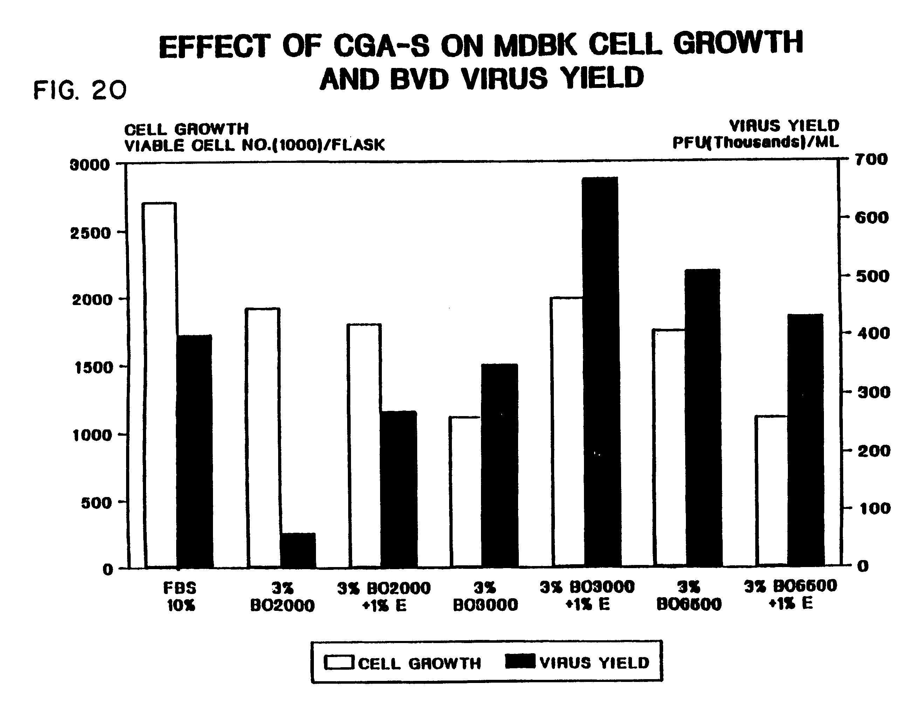

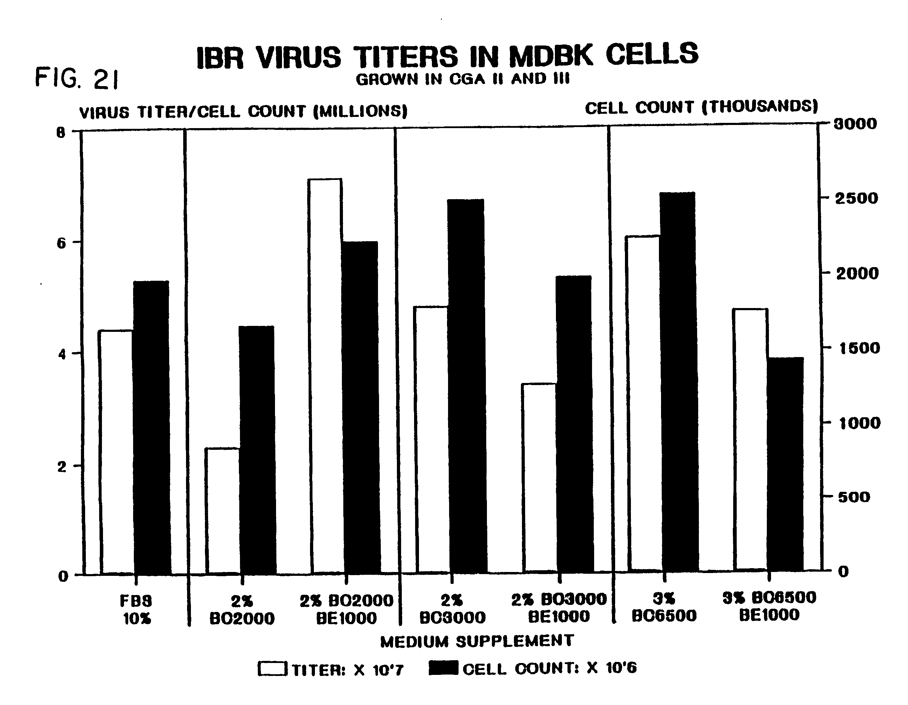

## Example 20 (Figs. 20 and 21)

CGA fractions of 3% BC3000 and 3% BC6500 appear to significantly enhance growth and BVD-virus production in MDBK cell cultures, comparing favorably with cultures grown in 10% FBS media (see Fig. 20). Similarly, IBR virus titres in MDBK cells (Fig. 21) are excellent in CGA enriched media as compared to 10% FBS, while cell growth of infected MDBK cells is particularly good in presence of 2% BE2000 plus 1% BC1000, 3% BC3000, 3% BC6500, or 3% BC6500 plus 1% BE1000.

Despite the apparent individual variation in the cultures with CGA media grown cells including virus-infected cells, the use of CGA preparations for growth and product enhancement consistently has been found to offer an improvement over FBS enriched media. The mean product yield enhancement is shown in Example 18.

## Example 21 (Fig. 22)

A number of GA III lots were tested for product enhancement effect on CHo-FCR cells. Productivity response in cells passaged in 1.5% CGA III plus 1% E passaged cultures was actually equal or superior to FBS (10%) passaged cultures when stimulated with CGA III + E at either 1.5% or 3.0% concentration.

The compositions containing the CGA fractions of the present invention enhance growth and productivity of cells and tissues in culture media. Similarly, compositions of CGA fractions may find efficacious use in the treatment of wounds for their potential wound healing effect.

The present invention also provides a pharmaceutical composition, which comprises a CGA fraction and a pharmaceutically acceptable carrier for topical use.

The dose of the CGA fraction used in the treatment of wounds, especially of the skin, will vary with the relative efficacy of the CGA material. However, as a general guide, suitable concentrations may be from about 1% to about 3% (v/v). Such amounts may be administered more than once a day, for example, several times a day, continuing over several days or weeks as long as such a treatment is needed.

Within the preferred range indicated above, the CGA preparation is not expected to have toxic effects. The preferred embodiment of the topical treatment of wounds is intended as utilizing CGA fraction derived from human serum or plasma for treatment of human skin. Similarly, in the case of other warm-blooded animals, the activators (CGA) of the invention are isolated from the identical species which is to be treated in order to avoid possible rejection or other immuno-toxicological effects.

For topical and percutaneous administration, the preparations may also be presented as an ointment, cream, lotion, gel, spray, aerosol, wash, skin paint or patch.

A composition of an effective amount of CGA fractions for topical or percutaneous use may also contain antioxidants, vitamins, amino acids, carbohydrates, lipids, trace metals, physiologically amenable salt solutions or buffers, minerals, as well as antibiotics and tissue activity modifying agents as tissue plasminogen activators or other factors.

## Claims

1. A method of isolating cell and tissue growth promoters from blood and platelet-free plasma comprising:

(a) removing blood cells and platelets from a sample of blood by centrifugation;

(b) incrementally separating growth activator-containing fractions by salt-precipitation;

(c) solubilizing the precipitated growth-promoter fractions followed by dialysis in a saline solution of physiological osmolarity; and

(d) pooling of the growth-supporting plasma for additive use in tissue and cell culture needs.

2. A method of producing cell and tissue growth promoting materials from blood fractions of plasma-derived cell-free sera comprising:

(a) centrifuging a sample of blood to remove blood cells and platelets and saving the supernatant liquid or plasma;

(b) inducing a clotting event by adding clot-forming activators to the supernatant plasma;

(c) removing the clot from the supernatant solution by centrifugation or decantation;

(d) purifying growth-promoting fractions from the supernatant solution by the salt precipitation steps as recited in claim 1; and

(e) pooling and packaging the purified growth promoting fractions for additive use in tissue and cell culture media.

3. The method of providing cell and tissue growth-promoting plasma fractions comprising:

(a) isolating plasma from a blood sample by removing the blood cells and platelets by centrifugation;

(b) separating the growth-promoting fractions as proteins from the plasma by salt-precipitation using about 30%, 50%, 60% and 70% saturated ammonium sulfate;

(c) solubilizing the salt-precipitated serum proteins at physiological concentrations; and

(d) pooling and packaging the solubilized fraction for additive use in tissue and cell culture media.

4. A fraction of serum or plasma containing cell and tissue growth-promoting properties comprising:

(a) serum or plasma proteins in physiological saline solution which were isolated from cell and platelet free blood liquids by precipitation with salt followed by solubilization and dialysis in physiological saline solution;

(b) said fraction being useful for additive use in tissue and cell culture media to promote the growth of tissues and cells therein; and

(c) said additive use also enhancing biological product yield.

5. The cell and tissue growth-promoting fraction as claimed in claim 4, wherein the cell growth-promoting activity is isolated in a fraction precipitated by about 50% saturated ammonium sulfate salt concentration.

6. The cell and tissue growth-promoting fraction as claimed in claim 4, wherein the cell growth-promoting fraction activity is isolated in a fraction precipitated by 10% to 30% saturated ammonium sulfate salt.

7. The cell and tissue growth-promoting fraction as claimed in claim 4, wherein the cell growth-promoting fraction activity is isolated in a supernatant fraction remaining after precipitation with about 50% saturated ammonium sulfate.

8. The cell and tissue growth-promoting fraction as claimed in claim 4, wherein the cell growth-promoting fraction activity is isolated in a fraction precipitated by 30% to 50% saturated ammonium sulfate salt.

9. The cell and tissue growth-promoting fraction as claimed in claim 4, wherein the dialyzed fraction is sterilized by filtration through a sterile filtration device.

10. The cell and tissue growth-promoting fraction as claimed in claim 4, which can be frozen and stored at about -20° centigrade or lower.

11. The cell and tissue growth-promoting fraction as claimed in claim 4, which can be stored as a lyophilized dry material and reconstituted in liquid form for effective use.

12. The cell and tissue growth-promoting fraction as claimed in claim 4, wherein the blood was harvested from ruminant animals.

13. The cell and tissue growth-promoting fraction as claimed in claim 4, wherein the blood was harvested from avian animals.

14. The cell and tissue growth-promoting fraction as claimed in claim 4, wherein the blood was harvested from human sources.

15. A salt-precipitated composition from blood having growth-promoting or product yield enhancing properties isolated by the steps comprising:

(a) providing for a supernatant plasma by centrifugation of a blood sample to remove cellular and platelet components;

(b) precipitation of a fraction of said supernatant plasma by mixing the supernatant plasma with enough ammonium sulfate to give a 30% saturated concentration;

(c) removal of the precipitated plasma fraction and saving the supernatant by centrifugation;

(d) dilution and dialysis of the precipitated plasma fraction in physiological saline solution; and

(e) isolation of further plasma fractions sequentially precipitated in 50% or 60% saturated ammonium sulfate using steps (c) and (d).

16. A composition of tissue and cell culture promoting nutrient media comprising in a liquid mixture aliquots of the growth-promoting fractions as claimed in claim 13, in media containing soluble mixture of amounts selected from the group consisting of vitamins, essential and non-essential amino acids, minerals, carbohydates, lipids, trace metals, buffers, sodium and potassium salts.

17. In a composition of tissue and cell culture nutrient media, the improvement comprising the addition of a tissue and cell growth promotion effective as well as cell product yield enhancing amount of the cell and tissue growth-promoting fraction as recited in claim 4.

18. In an improved tissue or cell culture nutrient media, wherein the improvement comprises replacing the natural product growth enhancer, such as fetal bovine serum, with a growth promoting serum or plasma fraction comprising serum or plasma proteins isolated from cell and platelet free blood liquids by precipitation with salt, in an amount sufficient to promote cell growth or product yield.

19. The improved tissue or cell culture nutrient media as recited in claim 18 which further comprises the addition thereto of a growth promotion or product yield effective amount of an Enhancer E, said Enhancer E produced from disrupted red blood cells by separating the enhancing supernatant from the cellular debris.

20. A pharmaceutical composition comprising a growth-promoting fraction as defined in claim 13 and a pharmaceutically acceptable carrier.

21. A method of topical or percutaneous treatment of warm-blooded animals including humans, which comprises administering to the sufferer an effective amount of a growth-promoting fraction as defined in claim 4 and a pharmaceutically acceptable carrier.

**Comparison of cell growth with fetal bovine serum and Biotis CGA**

FIG. I

No. Viable Cell/ml. x10$^5$

mg Protein/ml. Medium

CHO-K1 (Chinese hamster ovary)

C127I (Mouse mammary tumor)

HSBP (Human foreskin fibroblast)

MDCK (Canine [Dog] Kidney)

Protein mg Protein/ml. Medium

I Indicates standard deviation

10% (v/v) Fetal Bovine Serum

CGA-1

CGA-2

CGA-3

EP 0 396 402 A2

FIG. 2a CHO CELL PRODUCT YIELD

IN MEDIUM I

10% FBS    3% CGA + E

FIG. 2b CHO CELL PRODUCT YIELD

IN MEDIUM I

10% FBS    3% CGA + E

## FIG. 3a CHO CELL GROWTH

FIG. 3a CHO CELL GROWTH IN MEDIUM II

Legend: 10% FBS, 3% CGA + E

## FIG. 3b CHO CELL PRODUCT YIELD

FIG. 3b CHO CELL PRODUCT YIELD IN MEDIUM II

Legend: 10% FBS, 3% CGA + E

ivellement depose

# FIG. 4a FIBROBLAST CELL PRODUCT YIELD

IN MEDIUM I

CELL GROWTH: CPM (Thousands)

DAYS

10% FBS    3% CGA + E

# FIG. 4b FIBROBLAST CELL PRODUCT YIELD

IN MEDIUM I

PRODUCT YIELD: CPM (Thousands)

DAYS

10% FBS    3% CGA + E

## FIG. 5a  FIBROBLAST CELL PRODUCT YIELD

### IN MEDIUM II

CELL GROWTH: CPM (Thousands)

DAYS

10% FBS          3% CGA + E

## FIG. 5b  FIBROBLAST CELL PRODUCT YIELD

### IN MEDIUM II

PRODUCT YIELD: CPM (Thousands)

DAYS

10% FBS          3% CGA + E

FIG. 6a    B HYBRIDOMA PRODUCT YIELD

IN MEDIUM I

FIG. 6b    B HYBRIDOMA PRODUCT YIELD

IN MEDIUM I

## FIG. 7a B HYBRIDOMA PRODUCT YIELD

IN MEDIUM II

## FIG. 7b B HYBRIDOMA PRODUCT YIELD

IN MEDIUM II

FIG. 8a    T HYBRIDOMA CELL GROWTH

IN MEDIUM I

FIG. 8b    T HYBRIDOMA CELL GROWTH

IN MEDIUM II

FIG. 9

EFFECT OF MCDB 302, ALPHA MEM
AND ISCOVE'S MODIFIED DMEM MEDIA
ON THE GROWTH OF CHO-FCR CELLS

EP 0 396 402 A2

FIG. 10

EFFECT OF MCDB 302, ALPHA MEM
AND ISCOVE'S MODIFIED DMEM MEDIA
ON THE GROWTH OF CHO PLANT CELLS

MEAN VIABLE CELL NO./ML(X1000)

EP 0 396 402 A2

**FIG. 11**

EFFECT OF MCDB 302, ALPHA MEM
AND ISCOVE'S MODIFIED DMEM MEDIA
ON THE GROWTH OF CHO-KI

EP 0 396 402 A2

EP 0 396 402 A2

FIG. 12

## BEST MEDIUM AND CGA FOR CHO-K1 CELL

MEAN VIABLE OELL NO./ML(X1000)

**EFFECT OF MCDB 302, ALPHA MEM AND ISCOVE'S MODIFIED DMEM MEDIA ON THE GROWTH OF CHO-FCR CELLS**

FIG. 13

eingereicht / Newly filed
Nouvellement déposé

EP 0 396 402 A2

# CHO-K1 CELL GROWTH WITH CGA BC2000
## IN SUSPENSION CULTURE

FIG. 14

MEAN VIABLE CELL NO./ML.(MILLIONS)

DAY

M1 - WILLIAM'S MEDIUM E   M2 - MEM ALPHA MODIFICATION WITH RIBO AND DEOXYRIBONUCLEICACIDS

—✕— 10% FBS - M1      —△— 1% CGA+E - M1      —▽— 2% CGA+E - M1      —▲— 2% CGA+E - M2

—✕— 1% CGA+E - M1     —■— 1.5% CGA+E - M1    —+— 1% CGA+E - M1      —✚— 1% CGA+E - M2

ON THE DAY, INDICATED BY SYMBOL, PART OF THE MEDIUM WAS REPLACED WITH FRESH MEDIUM,
AND THE CELLS WERE COUNTED.

THE VIABILITIES WITH CGA WERE 97% OR BETTER.

IN 250ML SPINNER FLASKS WITHOUT
PH OR OXYGEN CONTROL.

EP 0 396 402 A2

Neu eingereicht / Newly filed
Nouvellement déposé

FIG. 15

EFFECT OF CELL GROWTH ACTIVATORS (CGA-S) AND FETAL BOVINE SERUM ON PRODUCT YIELD OF CHO-H$_1$ CELLS

FIG. 16

BEST MEDIUM AND CGA
FOR CHICK EMBRYO CELL

FIG. 17

CHICK EMBRYO CELL GROWTH IN ROLLER TUBE

EP 0 396 402 A2

TEST: LASHER

**EFFECT OF CGA-S ON THE CHICK EMBRYO CELL GROWTH AND THE SB-1 VIRUS YIELD**

FIG. 18

MEAN VIABLE CELLS

CELL NO. X $10^7$     VIRUS YIELD X $10^6$ PFU

EP 0 396 402 A2

TEST: LASHER

# EFFECT OF CGA-S ON THE CHICK EMBRYO CELL GROWTH AND THE HVT VIRUS YIELD

FIG. 19

MEAN VIABLE CELLS

FBS 5%

2%
BO 2000

2%
BC 3000

■ CELL NO. X $10^{7}$  ▨ VIRUS YIELD X $10^{6}$ PFU

EP 0 396 402 A2

# EFFECT OF CGA-S ON MDBK CELL GROWTH AND BVD VIRUS YIELD

**FIG. 20**

EP 0 396 402 A2

# IBR VIRUS TITERS IN MDBK CELLS
## GROWN IN CGA II AND III

FIG. 21

VIRUS TITER/CELL COUNT (MILLIONS)     CELL COUNT (THOUSANDS)

MEDIUM SUPPLEMENT

☐ TITER: X 10'7     ■ CELL COUNT: X 10'6

EP 0 396 402 A2

# MEAN PRODUCT YIELD ENHANCEMENT FOR 13 LOTS OF CGAIII WITH CHO-FCR CELLS PASSAGED IN 10% FBS OR 1.5% CGAIII + E

MEAN sFc/R YIELD: ug/ml

* A: PRODUCT EXPRESSED IN 1.5% CGAIII + E    B: PRODUCT EXPRESSED IN 6.0% CGAIII + E

BAR INDICATES STANDARD DEVIATION

## FIG. 22